# EUROPÄISCHE PATENTANMELDUNG

(11) **EP 3 208 213 A1**
(43) Veröffentlichungstag der Anmeldung: **23.08.2017**
(21) Anmeldenummer: 17000269.5
(22) Anmeldetag: 23.06.2015
(51) Int. Cl.: B65G 37/00, A61F 5/445, B31B 70/00

(54) **ANLAGE ZUM HERSTELLEN EINES MEDIZINPRODUKTS SOWIE VERFAHREN ZUM BETREIBEN EINER SOLCHEN ANLAGE**

(30) Priorität: 02.07.2014 DE 102014009682
(62) Teilanmeldung aus: 15001855.4
(71) Anmelder: Kiefel GmbH, 83395 Freilassing (DE)
(72) Erfinder: Haas, Johann, AT - 5203 Köstendorf (AT); Baumann, Jürgen, DE - 83339 Chieming (DE)
(74) Vertreter: Farago, Peter Andreas

(57) **Zusammenfassung**

Die Erfindung betrifft eine Anlage zum Herstellen eines Medizinprodukts, vor allem in Gestalt eines Ostomiebeutels, sowie ein Verfahren zum Betreiben einer solchen Anlage.

Konventionelle Anlagen führen verschiedene Folienanlagen in verschiedenen Ebenen im Abstand zueinander und ermöglichen dadurch Vorarbeitsschritte. Dann werden die Folien in einer Ebene zusammengeführt und miteinander verschweißt.

Die hier vorliegende Erfindung stellt ein hoch flexibles modulares Anlagenkonzept vor, bei welchem einzelne Zuführlinien für Halbzeuge vorgesehen sind. Die Zuführlinien können Halbzeuge vorkonfektioniert an eine Hauptlinie übergeben. In der Nauptlinie erfolgt unter größtmöglicher Arbeitspräzision das letztendliche Verarbeiten der Halbzeuge zum fertigen Produkt oder zu einem weiteren Halbzeug.

## Beschreibung

Die Erfindung betrifft eine Anlage zum Herstellen eines Medizinprodukts sowie ein Verfahren zum Betreiben einer solchen Anlage.

Zum Herstellen von Beuteln für medizinische Zwecke ist es bekannt, die erforderlichen Folienlagen in getrennten Ebenen voneinander zu führen und zum Verschweißen in eine Ebene zu bringen und im verschweißten Zustand in dieser Ebene weiter zu transportieren.

Dies gilt vor allem für ein Haupteinsatzgebiet der hier vorliegenden Erfindung, nämlich für medizinische Beutel, die eine Öffnung in einer Ober- oder Unterseite aufweisen, vor allem somit für jegliche Art von Ostomiebeuteln, vor allem Kolostomiebeutel.

Beispielsweise ist aus dem veröffentlichten technischen Datenblatt "Colostomy Bag Line KSM 102" der KIEFEL GmbH eine gattungsgemäße Anlage mit einer Hauptwerkzeuge aufweisenden Hauptlinie bekannt. Jedoch umfasst diese Anlage keine Zuführlinien.

Darüber hinaus ist aus der DE 21 60 708 A1 eine reine Fördervorrichtung zum Transportieren der von Schlauchmaschinen gefertigten Schlauchpakete zu Bodenmachermaschinen bekannt, wobei die Fördervorrichtung im Wesentlichen aus einer Anzahl von Band- und/oder Rollenförderern besteht, und wobei zwei zu Bodenmachermaschinen führende Förderriegel über einen Teil Ihrer Länge übereinander verlaufen, wobei die von zwei Schlauchmaschinen kommenden Förderwege durch Verstellen mindestens je einer Zwischenfördereinrichtung wechselweise an die beiden zu Bodenmachermaschinen führenden Förderwege anschließbar sind, und wobei mindestens einer der von dem Schlauchmaschinen kommenden Förderwege seitlich in die übereinander verlaufenden Förderwege eingeführt ist.

Die EP 0 045 587 A1 offenbart ferner eine Vorrichtung zur Herstellung von Ostomiebeuteln mit einer ersten Station mit einer Einrichtung zum Verschweißen zweier Beutelwände um einen Abschnitt ihres Umrisses, ferner mit einer zweiten Station, in welcher Einrichtungen vorgesehen sind, um nacheinander ein Trennelement in den teilweise umrissgeschweißten Beutel einzuführen, einen Klebezettel oder eine Frontplatte mit der von dem Trennelement gehaltenen Beutelwand zu verschweißen und das Trennelement herauszuziehen, und darüber hinaus mit einer dritten Station mit Einrichtungen zum Fertigstellen der Umrissschweißung.

Die DE 41 41 466 A1 offenbart eine Vorrichtung zur Durchführung eines Verfahrens zur Herstellung von vorzugsweise mit Seitenfalten versehenen Beuteln oder dergleichen, wobei sich die Vorrichtung auszeichnet durch ein taktweise antreibbares Förderband zur Aufnahme der teilweise vorgefertigten Beutel über auf diesem angeordnete Greifer, eine Zuführstation für die teilweise vorgefertigten Beutel, eine dieser in Förderrichtung des Förderbandes nachgeordnete Leimauftragsvorrichtung und eine auf diese folgende Zettelauftragsstation, welche in einem Abstand von der Zuführstation angeordnet ist, der ungefähr einer Vorzugslänge des Förderbandes entspricht.

Des Weiteren ist in der EP 1 227 052 A1 ein Verfahren und eine Einrichtung zum Umgreifen von mit Greifern gehaltenen geförderten, flachen Gegenständen beschrieben, welche einerseits einfacher ausgestaltet sind als entsprechende Verfahren bzw. Einrichtungen gemäß dem Stand der Technik und welche trotzdem deren Beschränkungen nicht aufweisen.

Der vorliegenden Erfindung liegt die Aufgabe zugrunde, dem Stand der Technik eine Alternative oder eine Verbesserung zur Seite zu stellen.

Nach einem ersten Aspekt der Erfindung löst diese Aufgabe eine Anlage zum Herstellen eines Medizinprodukts in Gestalt eines Ostomiebeutels, insbesondere eines Kolostomiebeutels, aus mehreren Lagen Folie und/oder Nonwoven, mit einem Halbzeugtransportsystem, insbesondere einem Tabletttransportsystem, mit einer Hauptlinie und mehreren seitlich angeordneten Zuführlinien, wobei Halbzeugzuführungen vorgesehen sind, um vorbearbeitete Halbzeuge von den Zuführlinien der Hauptlinie automatisiert zuzuführen, sowie mit Zuführlinienwerkzeugen an den Zuführlinien, und mit einem Hauptwerkzeug an der Hauptlinie, wobei die Halbzeugzuführungen modular am Tabletttransportsystem anordenbar sind.

Begrifflich sei hierzu Folgendes erläutert:
Zunächst sei ausdrücklich darauf hingewiesen, dass im Rahmen der hier vorliegenden Patentanmeldung unbestimmte Artikel und Zahlenangaben wie "ein", "zwei" usw. im Regelfall als Mindestens-Angaben zu verstehen sein sollen, also als "mindestens ein...", "mindestens zwei..." usw., sofern sich nicht aus dem jeweiligen Kontext ausdrücklich oder implizit ergibt oder es für den Fachmann erkennbar technisch zwingend ist, dass dort nur "genau ein...", "genau zwei..." usw. gemeint sein kann oder sein soll.

Außerdem sei daraufhingewiesen, dass wann immer von einer Steuerung oder Regelung die Rede ist, jeweils beide Begriffe offenbart sein sollen, wobei eine "Steuerung" als ein Oberbegriff zu einer "Regelung" verstanden sein soll.

Die hier vorgestellte Anlage stellt einen Ostomiebeutel her. Der Beutel selbst ist im frisch hergestellten, flachen Zustand ausgeprägt zweidimensional, also mit einer Oberseite und einer Unterseite versehen. Auf mindestens einer der beiden Seiten, also auf Oberseite und/oder Unterseite, ist bei einem Ostomiebeutel ein Ein- und/oder Ausgang vorgesehen.

Als eine "Folie" sei eine ausgeprägt zweidimensionale Struktur aus Kunststoff verstanden, insbesondere aus einem Thermoplasten. Als Varianten kommen nicht nur ein Nonwoven in Betracht, sondern beispielsweise auch ein Gestrick, Gelege, Gewebe oder Gewirk oder ein Vlies. Die Halbzeuge sollen jedoch in "Lagen" vorliegen.

Die Lagen können beispielsweise als fertige Abschnitte an die Anlage antransportiert werden, oder aber es ist beispielsweise eine Abwickelstation vorgesehen, auf welche eine Kaule gehängt werden kann, so dass sich die Anlage die einzelnen als Abschnitte erforderlichen Stücke der Lagen selbst von der Kaule abziehen und zuschneiden kann.

Die Lagen sollen früher oder später in der Anlage als "Abschnitte" diskreter Art vorliegen, somit als einzelnen Nutzen, wobei ein Nutzen bevorzugt nur einen Ostomiebeutel oder anderweitigen Beutel umfasst.

Das "Halbzeugtransportsystem" soll dazu eingerichtet sein, die Halbzeuge, basierend jeweils auf einem Abschnitt einer Lage, entlang der Zuführlinien und der Hauptlinie zu transportieren. Das Transportieren beinhaltet insbesondere das Zuführen zu Werkzeugstationen und das Weiterführen jenseits der Werkzeugstationen.

Bei einem "Tabletttransportsystem" sind einzelne, diskrete Tabletts vorgesehen, welche die Abschnitte oder die daraus hergestellten Halbzeuge transportieren. Die Tabletts selbst können beispielsweise von einem Bandantrieb, einem magnetischen Antrieb, einem Mitnehmerantrieb oder von beliebigen anderen Antrieben durch die Anlage transportiert werden.

Die Maschinenrichtung bei einer solchen Anlage ist aufgefächert, nämlich bei den Zuführlinien hin zur Hauptlinie, und in der Hauptlinie von einer ersten Zuführlinie hin zum Ausgabepunkt aus der Anlage, idealerweise mit dem fertigen Produkt.

Das Halbzeugtransportsystem weist bevorzugte diskrete Transportmittel für jeweils einen Abschnitt der Lagen auf.

Die "Halbzeugzuführung" soll dazu eingerichtet sein, vollautomatisiert die vorbearbeiteten Halbzeuge zu übernehmen, wenn sie von den Zuführlinien kommen, und jeweils von einer Zuführlinie auf die Hauptlinie zu transferieren.

An den Zuführlinien sollen Zuführlinienwerkzeuge vorgesehen sein, also mindestens zwei Zuführlinien sollen vorgesehen sein, welche jeweils mindestens ein Werkzeug aufweisen.

Die Hauptlinie soll mindestens ein Hauptwerkzeug aufweisen.

Vorteilhaft erreicht der erste Aspekt der Erfindung eine sehr hohe Variabilität in der Produktion der Ostomiebeutel: Durch die Halbzeugzuführungen, welche jeweils als Transfereinrichtung zwischen einer Zuführlinie und der Hauptlinie dienen, ist es möglich, eine oder mehrere, mitunter auch alle, Zuführlinien auszutauschen, wenn beispielsweise ein Produktwechsel durchgeführt werden soll. Teile der Anlage können dennoch verbleiben. Wenn die Zuführlinien mit einem ausreichenden Abstand zueinander seitlich angeordnet sind, lassen sich die Zuführlinien beispielsweise einfach seitlich, somit parallel zur Hauptlinie, verschieben, also zwischen einer Ruheposition und einer Arbeitsposition, in welcher sie an der Hauptlinie angeschlossen sind. Bei einer solchen Konstellation lässt sich somit ein Produktwechsel ohne jeden Platzverlust durchführen.

Die Halbzeugzuführungen sind hierbei modular am Tabletttransportsystem anordenbar.

Nach einem zweiten nicht erfindungsgemäßen Aspekt ist eine Anlage zum Herstellen eines Medizinprodukts, vor allem eines Beutels, aus mehreren Lagen flächiger Abschnitte, insbesondere Folie und/oder Nonwoven aufweisend, mit einem Halbzeugtransportsystem, insbesondere einem Tabletttransportsystem, mit einer Hauptlinie und mehreren seitlich angeordneten Zuführlinien, wobei Halbzeugzuführungen vorgesehen sind, um vorbearbeitete Halbzeuge von den Zuführlinien der Hauptlinie automatisiert zuzuführen, mit Zuführlinienwerkzeugen an den Zuführlinien, und mit einem Hauptwerkzeug an der Hauptlinie vorteilhaft, wobei die Zuführlinien und die Hauptlinie dieselbe Arbeitshöhe aufweisen.

Unter der "Arbeitshöhe" sei diejenige Höhe verstanden, auf welcher die Abschnitte der Lagen in mindestens einem Werkzeug mindestens einer Zuführlinie und der Hauptlinie geführt werden, bezogen auf einen horizontalen Arbeitsboden, auf welchem die Anlage aufgestellt ist.

Mit einfachen Worten sieht der zweite Aspekt der Erfindung vor, dass möglichst viele, jedenfalls aber einige Arbeitsschritte auf derselben Arbeitshöhe passieren.

Auf diese Weise muss die Halbzeugzuführung nicht in der Höhe justiert werden, wenn zwei Zuführlinien ausgewechselt werden, beispielsweise bei einem Produktwechsel. Besonders vorteilhaft ist es, wenn die Zuführlinien an der Übergabestation zur Transfereinrichtung, also zur Halbzeugzuführung, die gleiche Arbeitshöhe aufweisen, wie in der Hauptlinie das jeweils folgende Werkzeug nach der Zuführung aufweist.

Die bevorzugte Ausführungsform der Anlage sieht vor, dass sämtliche Werkzeuge der Hauptlinie, jedenfalls soweit in diesem Abschnitt noch Zuführlinien angeschlossen sind, sowie die jeweils zugehörigen Endpositionen der Zuführlinien, auf selber Arbeitshöhe liegen, vor allem jedoch alle.

Nach einem dritten nicht erfindungsgemäßen Aspekt ist eine Anlage zum Herstellen eines Medizinprodukts, vor allem eines Beutels, aus mehreren Lagen flächiger Abschnitte, insbesondere Folie und/oder Nonwoven aufweisend, mit einem Halbzeugtransportsystem, insbesondere einem Tabletttransportsystem, mit einer Hauptlinie und mehreren seitlich angeordneten Zuführlinien, wobei Halbzeugzuführungen vorgesehen sind, um vorbearbeitete Halbzeuge von den Zuführlinien der Hauptlinie automatisiert zuzuführen, mit Zuführlinienwerkzeugen an den Zuführlinien, und mit einem Hauptwerkzeug an der Hauptlinie vorteilhaft, wobei die Halbzeugzuführung eine Spreizeinrichtung für das zuzuführende Halbzeug aufweist.

Der dritte Aspekt der vorliegenden Erfindung sieht mit einfachen Worten vor, dass die Transfereinrichtung, also die Halbzeugzuführung, das Halbzeug, somit den Abschnitt der herzustellenden Lage, spreizt.

Das Spreizen soll zumindest temporär erfolgen.

Bevorzugt jedoch wird das Halbzeug in gespreiztem Zustand auf die Hauptlinie übergeben.

Unter einem "Spreizen" wird verstanden, dass das Halbzeug in mindestens einer seiner zwei Dimensionen elastisch oder plastisch gedehnt wird. Bei der Übergabe auf die Hauptlinie wird die Dehnung bevorzugt fixiert.

Von Vorteil erreicht der dritte Aspekt der Erfindung, dass etwaige Durchhänge oder Wellen gestrafft werden, so dass die Hauptlinie unabhängig von solchen verarbeitungsbedingten Unpräzisionen arbeiten kann. Die Qualität des Produkts wird dadurch besser.

Ausdrücklich sei betont, dass zwei der drei vorstehend eingeführten Erfindungsaspekte in beliebiger Kombination auch gleichzeitig anwendbar sind, wodurch nochmals Synergieeffekte entstehen. Auch alle drei vorstehend genannten Aspekte können gleichzeitig verwirklicht sein.

An der Hauptlinie ist bevorzugt ein Hauptschweißwerkzeug vorgesehen.

Ein Hauptschweißwerkzeug ist dazu vorgesehen, die verschiedenen Halbzeuge miteinander zum letztendlich mehrlagigen Medizinprodukt, vor allem Beutel, zu verbinden. Vorher können schon Teilverbindungen vorgesehen sein, entweder auf der Hauptlinie oder auch auf den Zuführlinien.

Eine Zuführlinie weist bevorzugt ein Vorschweißwerkzeug auf, vor allem können mehrere Zuführlinien jeweils ein Vorschweißwerkzeug aufweisen. Vorschweißungen können dadurch schon vor dem Transfer der Halbzeuge auf die Hauptlinie erfolgen.

Die Zuführlinien können alternativ oder kumulativ ein Vorstanzwerkzeug oder mehrere Vorstanzwerkzeuge oder andere Trennwerkzeuge aufweisen.

Sowohl die Zuführlinien als auch die Hauptlinie können außerdem ein kombiniertes Schweiß-/Trennwerkzeug aufweisen.

Wenn die Halbzeugzuführung voneinander motorisch entfernbare Greifer aufweist, fällt es besonders leicht, das Halbzeug in der Zuführung zur Hauptlinie zu spreizen.

Bevorzugt ist eine Steuerung vorgesehen, in welcher das Maß der Spreizung einstellbar ist.

Es wird vorgeschlagen, dass die Halbzeugzuführung vier Greifer aufweist, vor allem genau vier Greifer.

Die Abschnitte der Lagen, somit die Halbzeuge, werden in der Praxis besonders gut handhabbar sein, wenn sie zumindest im Wesentlichen eine Rechteckform haben. Bei einer Mehrzahl von Greifern wird deshalb vorgeschlagen, dass die Greifer an jeweils einer Ecke des Halbzeugs greifen, somit bei einem zumindest im Wesentlichen rechteckigen Halbzeug genau vier Greifer jeweils genau eine Ecke des Halbzeugs greifen. Die Greifer können dann besonders leicht in einer oder in zwei Richtungen eine Spreizung des Halbzeugs vornehmen.

Wenn die Halbzeugzuführung Greifer in Form von Nadeln aufweist, dann kann das Halbzeug in bewährter Weise durch einen einfachen Einstich der Nadel, bevorzugt im Bereich der Ecke, eingenahtet werden. Es ist dann besonders leicht, das Halbzeug präzise zur Hauptlinie zu übergeben.

Um den gespreizten Zustand des Halbzeugs an der Hauptlinie zu erhalten, wird vorgeschlagen, dass die Halbzeugzuführung Ablagemittel für das Halbzeug in gespanntem Zustand aufweist, um diese auf Spannungshaltern der Hauptlinie am Halbzeugtransportsystem zu platzieren.

Als "in gespanntem Zustand" sei auch ein relaxierter Zustand verstanden, der sich nach einem ursprünglich gespannten Zustand einstellen kann, beispielsweise wenn der Kunststoff noch warm ist.

Es wurde bereits erwähnt, dass bevorzugt alle Halbzeugzuführungen auf identischer Höhe zugreifen und/oder abzulegen eingerichtet sein können.

Wenn die Anlage Tabletts in Maschinenrichtung auf genau einer Ebene zu führen eingerichtet ist, wird ebenfalls die Übergabe der Halbzeuge von den Zuführlinien zur Hauptlinie erleichtert.

Außerdem fällt es besonders leicht, eine zweite Ebene zu definieren, in welcher die Tabletts rückgeführt werden, um ein bevorzugtes endlos umlaufendes System zu ergeben.

Zwischen zwei Halbzeugzuführungen an der Hauptlinie kann bevorzugt eine Warteposition vorgesehen sein.

Dies bietet sich vor allem dann an, wenn die Transportmittel des Halbzeugtransportsystems diskret sind, beispielsweise Tabletts. Insbesondere sollten die einzelnen Transportmittel individuell ansteuerbar sein. So wird es möglich, die Arbeitstakte verschiedener Zuführlinien und/oder der Hauptlinie voneinander zu entkoppeln.

Es ist dann denkbar, Tabletts mit jeweils einem oder mehreren Halbzeugen von der Halbzeugzuführung zur Hauptlinie übergeben zu lassen, oder die darauf angeordneten Halbzeuge von einem Tablett zu übernehmen, welches sich nur an der Zuführlinie befindet, und auf ein Tablett abzulegen, welches sich nur an der Hauptlinie befindet.

Es wird als vorteilhaft empfunden, wenn die Halbzeugzuführung eine Schlechtteilauszweigung aufweist.

Dafür ist bevorzugt ein Sensor vorgesehen, vor allem ein optischer und/oder mechanischer Sensor. Ein Controller kann dann erkennen, ob aus der Zuführlinie ein Schlechtteil kommt. Dieses kann von der Halbzeugzuführung - anstelle in die Hauptlinie eingebracht zu werden - in einen Ausschusssammler gebracht werden. Die Halbzeugzuführung eignet sich besonders, weil sie ohnehin eine Greifeinrichtung für die Halbzeuge am Ende der Zuführlinie aufweist.

Die Hauptlinie kann eine lineare Transportrichtung aufweisen. Bei einer solchen Transportrichtung sind die Zuführlinien bevorzugt im selben Winkel, insbesondere orthogonal zur Maschinenrichtung der Hauptlinie, angeordnet,

Alternativ lassen sich verschiedene andere Transportrichtungen denken, beispielsweise ovale Transportrichtungen, oder es kann ein großer Schaltrevolver sein, der um einen Schaltwinkel geschaltet wird und dabei den Transport bewirkt.

Um einen medizinischen Beutel herzustellen, ist es vor allem denkbar, dass mindestens vier Lagen, vor allem genau vier Lagen, zum Herstellen des Produkts miteinander verbunden werden.

Das Halbzeugtransportsystem kann mit den Halbzeugzuführungen gleichgetaktet sein.

Nach einem vierten Aspekt der vorliegenden Erfindung löst die gestellte Aufgabe ein Verfahren zum Betreiben einer Anlage wie vorstehend beschrieben.

Diese Anlage wird idealerweise dazu betrieben, ein Medizinprodukt herzustellen.

Wenn ein Produktwechsel während der Produktion vorgenommen werden soll, kann dies einfach dadurch bewerkstelligt werden, dass ein oder mehrere Zuführlinien ausgetauscht werden.

Die Erfindung wird nachstehend anhand von Ausführungsbeispielen unter Bezugnahme auf die Zeichnungen näher erläutert. Dort zeigen
- Fig. 1: schematisch in einer perspektivischen Ansicht eine Anlage zum Herstellen von Kolostomiebeuteln, mit einer Hauptlinie, mit drei Zuführlinien und mit einer Fertigproduktlinie,
- Fig. 2: schematisch in einer Detailansicht aus Figur 1 eine mögliche erste Zuführlinie,
- Fig. 3: schematisch in einer Detailansicht aus Figur 1 eine mögliche zweite Zuführlinie,
- Fig. 4: schematisch in einer Detailansicht aus Figur 1 eine mögliche dritte Zuführlinie,
- Fig. 5: schematisch in einer Detailansicht die Hauptlinie,
- Fig. 6: schematisch in einem Detail aus Figur 1 ein Ende der Hauptlinie mit einer Gutteilentahme und einem Gutteiltisch,
- Fig. 7: in perspektivischer Ansicht eine Transfereinrichtung von einer Zuführlinie zur Hauptlinie mit einem gegriffenen Halbzeug aus Folie,
- Fig. 8: die Transfereinrichtung aus Figur 8 aus einer geänderten Perspektive sowie
- Fig. 9: die Transfereinrichtung aus den Figuren 7 und 8

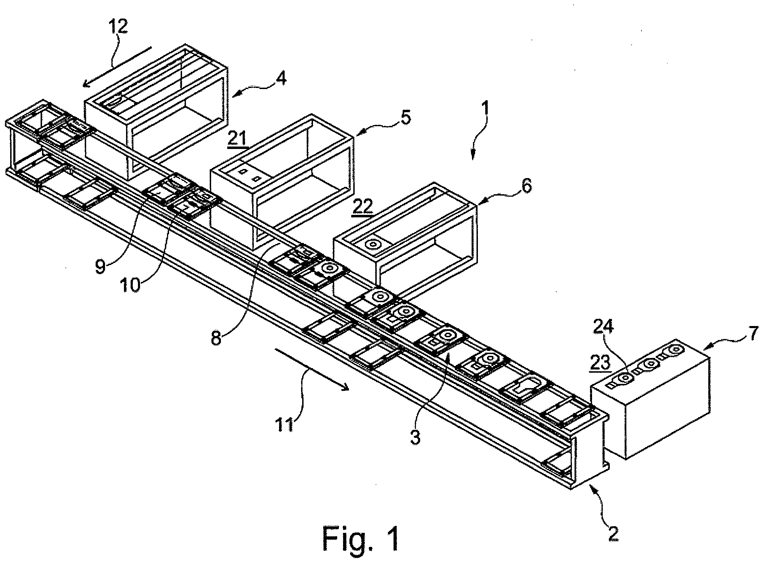

Die Anlage 1 in Fig. 1 ist eine modular zusammengesetzte Anlage zum Herstellen von Kolostomiebeuteln. Ausdrücklich sei darauf hingewiesen, dass auch verschiedenste andere mehrlagige Produkte in einer solchen Anlage vorteilhaft hergestellt werden können.

Die Anlage 1 besteht im Wesentlichen aus einer Hauptlinie 2 mit einem Tabletttransportsystem 3 sowie mit einer ersten Zuführlinie 4, einer zweiten Zuführlinie 5 und einer dritten Zuführlinie 6 sowie mit einer ebenfalls als seitliche Linie ausgestalteten Fertigteilübernahme 7.

Das Tabletttransportsystem 3 in der Hauptlinie 2 besteht aus einer linearen Führung 8, in welcher eine Vielzahl Tabletts 9 (exemplarisch beziffert) längsverschieblich und - bevorzugt individuell - angetrieben geführt sind.

Jedes Tablett 9 hat eine rechteckige Auflagefläche 10 zur Aufnahme von Halbzeugen oder fertigen Produkten.

Die Tabletts 9 sind in einer Maschinenrichtung 11 zu transportieren eingerichtet.

Im hier vorgestellten, konkreten Ausführungsbeispiel führt eine darunterliegende Ebene die Tabletts in einem endlosen Umlauf in der Hauptlinie 2 wieder gegen die Maschinenrichtung 11 zurück.

Seitlich der Hauptlinie 2 stehen die drei Zuführlinien 4, 5, 6, hier in einem orthogonalen Winkel, so dass die Hauptlinie 2 in der Haupt-Maschinenrichtung 11 durch die Anlage 1 transportiert, während die Zuführlinien 4, 5, 6 in Zuführ-Maschinenrichtungen 12 (exemplarisch dargestellt) Halbzeuge zur Hauptlinie zuführen.

Die Zuführlinien 4, 5, 6 verfügen über Werkzeuge für unterschiedliche Vorbearbeitungen.

Die Zuführlinien 4, 5, 6 und die Hauptlinie 2 verfügen außerdem jeweils über eine Arbeitshöhe, wobei die Arbeitshöhen sowohl in den Zuführlinien 4, 5, 6 als auch in der Hauptlinie identisch sind.

Von jeder Zuführlinie 4, 5, 6 gibt es eine Transfereinrichtung (nicht dargestellt), welche dazu eingerichtet ist, in einer Zuführ-Maschinenrichtung antransportiert werdende Halbzeuge von den Zuführlinien 4, 5, 6 zu übernehmen und auf die Tabletts 9 in der Hauptlinie 2 zu platzieren.

Die Werkzeuge sind hier nicht dargestellt.

Im Regelfall wird die Hauptlinie 2 jedenfalls über ein Hauptschweißwerkzeug verfügen (ebenfalls nicht dargestellt).

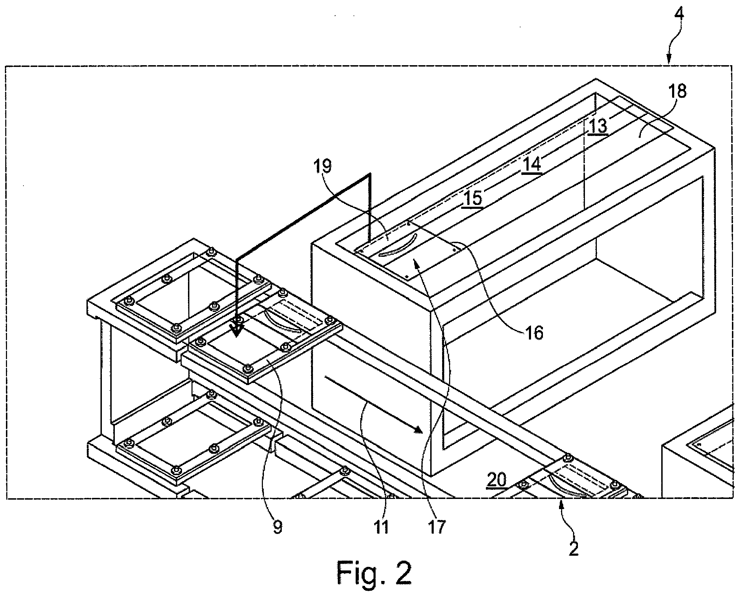

Die erste Zuführlinie 4, oder auch jede andere Zuführlinie, kann beispielsweise wie folgt aufgebaut sein (vgl. Fig. 2):

Zunächst findet an einer ersten Position 13 das Abwickeln einer Folie von einer Rolle statt.

In einer folgenden Position 14 findet eine Vorverschweißung statt.

In einer weiterfolgenden Position 15 erfolgt eine Stanzung oder Schneidung.

Es kann auch ein Trennschnitt 16 kombiniert oder separat an einer Station erfolgen. Der Trennschnitt 16 separiert einen Folienabschnitt 17 vom zulaufenden Endlosfolienband 18.

Der Folienabschnitt 17 ist hier rechteckig belassen. Die Form des Folienabschnitts 17 korrespondiert insoweit prinzipiell mit derjenigen des Tabletts 9.

Ebenfalls bereits in der Zuführlinie 4 werden dem Folienabschnitt 17 vier kleine Löcher 19 (exemplarisch beziffert) gestanzt, und zwar genau in den Ecken des rechteckigen Folienabschnitts 17.

Eine Halbzeugzuführung (nicht dargestellt) führt einen Transfer der Halbzeuge in Form der nun vereinzelten Folienabschnitte 17 von der Zuführlinie 4 zur Hauptlinie 2 durch, konkret durch Greifen des Folienabschnitts 17, Anheben des Folienabschnitts 17, Vorwärtsbewegung des Folienabschnitts 17, dabei Drehung um 90°, und Ablage des Folienabschnitts 17 auf das Tablett 9. Ob ein Folienabschnitt 17 oder ein anderes Halbzeug beim Transfer gedreht wird, wird immer im Einzelfall von der Form der zulaufenden Halbzeuge und der Form der Transportmittel abhängig gemacht werden.

Das Tablett 9 kann anschließend mit der Maschinenrichtung 11 weiter entlang der Hauptlinie 2 transportiert werden.

Gleichzeitig können entlang der Zuführlinie 4 weitere Halbzeuge vorproduziert werden und auf weitere Tabletts in der Hauptlinie 2 transferiert werden.

Weil Abstände 20 zwischen den einzelnen Tabletts 9 in der Hauptlinie 2 belassen sind, muss der Produktionstakt der Zuführlinie 4 nicht mit dem Vorschubtakt in der Hauptlinie 2 übereinstimmen.

Vielmehr ist es auch möglich, vor oder während des Transfers von der Zuführlinie 4 zur Hauptlinie 2 eine Gutteilerkennung beziehungsweise eine Schlechtteilabführung vorzusehen.

Die Tabletts 9 können vollflächig oder teilflächig genutzt werden.

Ausdrücklich sei außerdem darauf hingewiesen, dass das hier dargestellte Ausführungsbeispiel eine Tablettanlage ist. Es können aber auch andere Anlagengeometrien oder Transportarten vorgesehen sein, beispielsweise kann die Anlage als Drehtischanlage oder als Ovaltransportanlage ausgeführt sein.

Dadurch, dass nur eine einzige Folienebene existiert, also die Arbeitshöhen der zulaufenden Folienbahn in der Zuführlinie, der Folienabschnitte und der Tabletts identisch sind, können die einzelnen Zuführlinien 4, 5, 6 modular an die Hauptlinie 2 angesetzt werden und somit auch untereinander ausgetauscht oder durch weitere, nicht dargestellte Zuführlinien ersetzt werden, beispielsweise um einen Produktwechsel durchzuführen.

Die Produktionsgenauigkeit wird durch diese Maßnahmen drastisch erhöht.

Außerdem sind in der Hauptlinie 2, somit in der Linie der finalen Bearbeitungsschritte wie beispielsweise der Hauptverschweißung, keinerlei Zugspannungen auf der Folie, welche aus einem langen Folienband herrühren könnten, weil bereits vorher, nämlich in den Zuführlinien 4, 5, 6, eine Vereinzelung des Endlosfolienbandes 18 zu Folienabschnitten 17 erfolgt.

Auch im Falle eines gewünschten Umbaus der Anlage 1 zum Herstellen eines neuen Produkts ist dies leichter möglich als im Stand der Technik bekannt: Denn es können weitere als Zuführlinien dienende Module separat vorkonfektioniert werden und dann vom Maschinenbauer zum Beutelproduzenten transportiert werden. Wegen der einheitlichen Übergabe-Schnittstelle von den Zuführlinie 4, 5, 6 zur Hauptlinie 2 können solche nachträglich konstruierten, modularen Zuführlinien jederzeit nachgerüstet werden.

Um mit vorhandenen Modulen schnell die Anlage 1 umrüsten zu können, können die Module beispielsweise in Zwischenräume 21, 22 bewegt werden, somit parallel zur Hauptlinie 2 und rechtwinklig zur Zuführ-Maschinenrichtung 12.

So kann beispielsweise eine lineare Lagerung für die Zuführlinien 4, 5, 6 an der Hauptlinie 2 vorgesehen sein, um die modularen Zuführlinien 4, 5, 6 in ihrem Abstand zur Hauptlinie 2 eindeutig festzulegen.

Am Ende 23 der Hauptlinie 2 ist eine Entnahmevorrichtung (nicht dargestellt) für die fertigen Produkte, hier Ostomiebeutel 24 (exemplarisch beziffert), vorgesehen. Die Entnahmeeinrichtung kann bevorzugt zwischen Gutteilen und Schlechtteilen unterscheiden und die Schlechtteile dem Ausschuss zuführen, während die Gutteile der Ostomiebeutel 24 auf die Fertigteilübernahme 7 abgelegt werden und von dort weitertransportiert werden können.

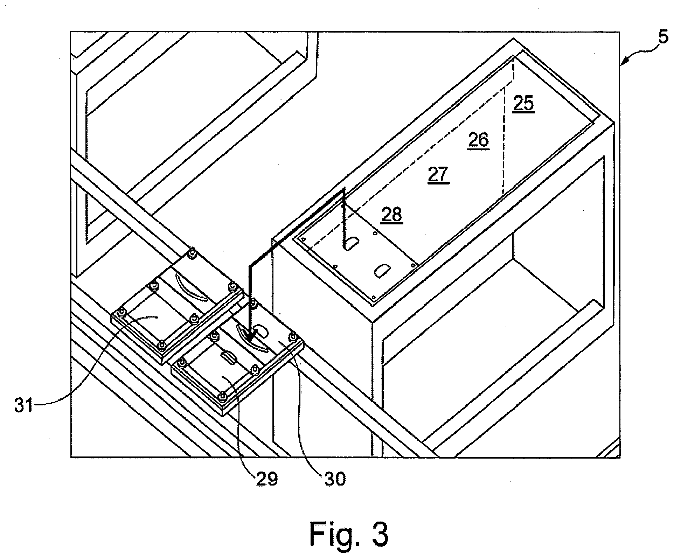

Die zweite Zuführlinie 5 kann insbesondere so konfiguriert sein (vgl. Fig. 3), dass ebenfalls zunächst eine Folienabwicklung 25 stattfindet, daraufhin ein Filter ausgestanzt und eine Vorschweißung vorgenommen wird (Position 26). Anschließend kann eine Klappe angebracht werden (Station 27). Schließlich können wieder Löcher im Eckbereich angebracht werden, und es kann ein Trennschnitt vom endlosen Folienband zu einem Einzelnutzen erfolgen (Station 28). Anschließend kann der Transfer auf ein bereitstehendes Tablett 29 erfolgen, wobei das bereitstehende Tablett 29 bereits ein Halbzeug 30 aus einer vorherigen Zuführlinie, hier der ersten Zuführlinie 4, trägt.

Durch die bereits in der Zuführlinie eingebrachten Löcher im Eckbereich lassen sich die verschiedenen, von den Zuführlinien 4, 5, 6 transferiert werdenden Halbzeuge 30 perfekt übereinander ausgerichtet platzieren.

Währenddessen können in Warteposition befindliche Tabletts 31 stehen oder individuell Bewegungen durchführen, ohne den Transfer an der zweiten Zuführlinie 5 oder der ersten Zuführlinie 4 zu bremsen.

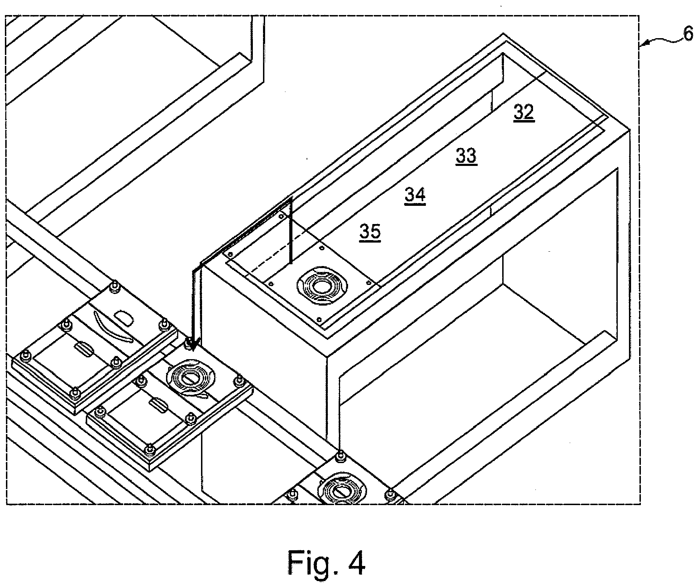

Eine weitere Zuführlinie, hier beispielsweise die dritte Zuführlinie 6, kann wie folgt aufgebaut sein (vgl. Fig. 4):
Zunächst erfolgt wieder das Abwickeln einer Folienbahn (Station 32). Anschließend kann ein Eingangs- und/oder Ausgangsloch gestanzt oder anderweitig herausgetrennt werden (Station 33). Ein Flansch kann zugeführt und angeschweißt werden (Station 34). Schließlich wird bevorzugt wiederum ein Trennschnitt durchgeführt und Zentrierungs- und Spannlöcher an den Ecken eingebracht (Station 35). Der anschließende Transfer positioniert wiederum das frisch zugeführte Halbzeug auf das bereits auf dem Tablett antransportierte Halbzeug.

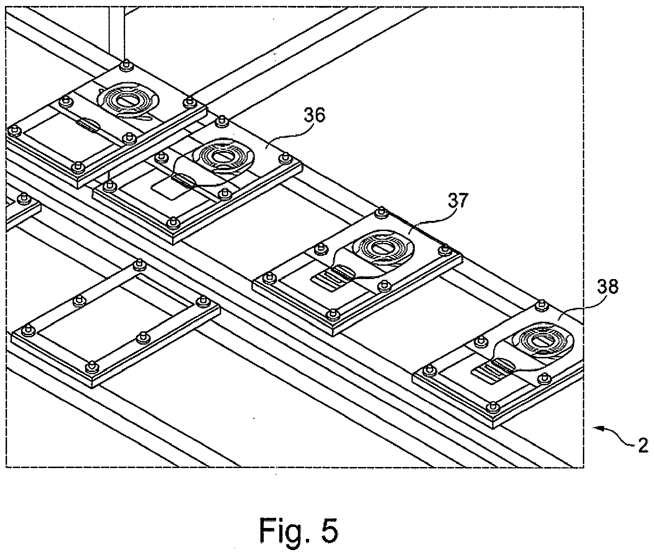

In der Hauptlinie 2 können vor der ersten Zuführlinie 4, nach der letzten, hier dritten, Zuführlinie 6 sowie zwischen den Zuführlinien 4, 5, 6 weitere Stationen mit Bearbeitungswerkzeugen vorgesehen sein, so beispielsweise eine Station 36 mit einem Umfangsschweißwerkzeug für den Beutel, eine Station 37 zum Anbringen eines Kennzeichnungslabels oder zum Bedrucken sowie beispielsweise eine weitere Station 38 zur Qualitätskontrolle, beispielsweise durch eine optische Kontrolleinrichtung (vgl. Fig. 5).

Am Ende 23 der Hauptlinie erfolgt sodann bevorzugt ein Ausstanzen an einer Station 39 sowie ein Ausreißen oder anderweitiges Entfernen an einer Station 40 sowie schließlich eine Entnahme 41 von Gutteilen sowie eine Entfernung von Schlechtteilen.

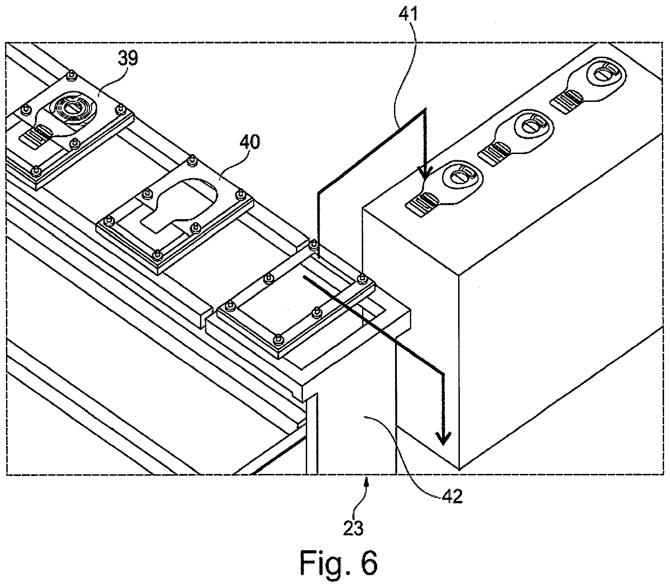

Ein Lift 42 dient dazu, die Tabletts 9 aus der Arbeitshöhe der Hauptlinie 2 in eine Rücktransport-Höhe abzusenken (vgl. Fig. 6).

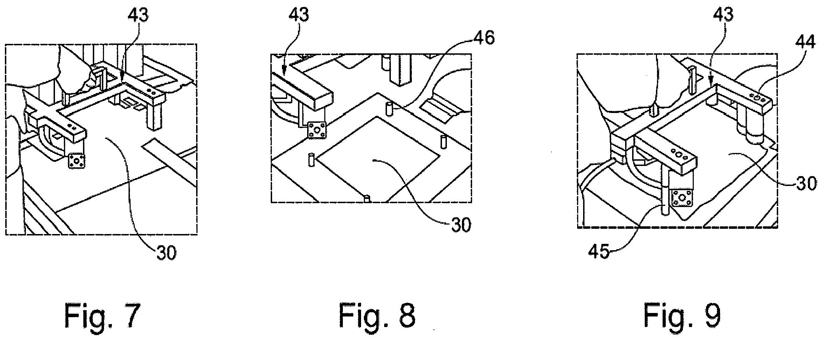

Die Halbzeugzuführung (vgl. Fig. 7 bis 9) verfügt über einen komplexen Greifer 43.

Die Greifeinrichtung 43 ist schwimmend an der Halbzeugzuführung gelagert, er wird über einen Sensor zentriert, wobei der Sensor beispielsweise optisch sein kann, aber auch mechanisch.

Alternativ lässt sich gut vorstellen, dass die Tabletts schwimmend gelagert sind.

Auch lässt sich vorstellen, dass das Tablett zum Ablegen des Halbzeugs auf das Tablett aus seiner Transportebene herausgehoben und zentriert wird.

Die Greifeinrichtung 43 verfügt über mehrere Greifer 44, 45 (exemplarisch beziffert), insbesondere über genau vier Greifer 44, 45.

Die Greifer 44, 45 sind bevorzugt an der Greifeinrichtung 43 so eingestellt oder so vorverfahren, dass sie an die Ecken des zugreifenden Halbzeugs 30 greifen können, insbesondere in die dort vorgesehenen Löcher 19, oder sich relativ zu den Löchern 19 ausrichten, wiederum beispielsweise mechanisch und/oder optisch.

Die Greifer 44, 45 können bevorzugt relativ zueinander elektromotorisch verstellt werden, so dass auf das Halbzeug 30 eine Zugspannung aufgebracht werden kann. Dies verhindert einen Durchhang, wobei selbst schon bestehende Falten geglättet werden können, so dass das Halbzeug 30 in möglichst glattem Zustand auf Zentrierpins 46 (exemplarisch beziffert) am bereitstehenden Tablett 29 aufgelegt werden können.

Alternativ oder kumulativ lässt sich vorstellen, dass Nadelgreifer vorgesehen sind, welche die Zuschnitte in der Hauptlinie 2 aufnehmen.

Es sei ausdrücklich darauf hingewiesen, dass bereits das Maschinenkonzept zum Herstellen eines mehrlagigen Medizinprodukts eine Erfindung darstellt, nicht zwingend nur eingeschränkt auf eine Herstellanlage für Ostomiebeutel.

Die Herstellung von Ostomiebeuteln ist eine vorteilhafte Ausgestaltung der Erfindung.

Alle übrigen Werkzeuge an den Zuführlinien und der Hauptlinie wird der Fachmann im Rahmen der Konstruktion einer Anlage zum Herstellen eines bestimmten Medizinprodukts so wählen, wie es ihm gerade geschickt erscheint, beispielsweise mit separierten Werkzeugen an separierten Stationen, oder aber mit kombinierten Werkzeugen an kombinierten Stationen, so dass beispielsweise auch ein kombiniertes Schweiß-SchneidWerkzeug vorgesehen sein kann, anstelle von einer einzelnen Station zum Vorschweißen oder Hauptschweißen und einer davon separaten Station zum Herausschneiden oder anderweitigen Heraustrennen.

Generell lässt sich die hier vorgestellte Erfindung am vorteilhaftesten anwenden für Medizinprodukte mit mehreren Folienlagen und einem Hauptschweißvorgang sowie mit einem Hauptschweißvorgang vorgelagerten Operationen, wobei insbesondere auch an die Folie Gegenstände angebracht werden können, beispielsweise ein Flansch oder eine Klappe.

Bei konventionellen Anlagen werden beispielsweise vier Folienanlagen in vier Ebenen im Abstand zueinander geführt, um Aufgaben wie Stanzen, Filtervorschweißung etc. zu ermöglichen. Erst dann werden die Folien in einer Ebene zusammengeführt und miteinander verschweißt.

Bei Produktänderungen ist bei diesem Konzept jedoch der Produktwechsel eingeschränkt, und jeder Folgeschritt steht in Abhängigkeit zum vorgelagerten Schritt.

Bei dem neuen Konzept sind die Bearbeitungsschritte aufgeteilt in die zuführenden Module, und fertig vorgearbeitete (beispielsweise vorgestanzte oder vorgeschweißte Zuschnitte) werden am Tablett gesammelt und dann erst final verschweißt.

Eine weitere Besonderheit ist die Spreizmöglichkeit der z.B. vier in den Ecken montierten Nadelgreifern. Dadurch wird Durchhang vermieden, und etwaige Falten werden geglättet.

Erreicht wird die Herstellung eines Produkts aus mehreren Lagen Folie und/oder Nonwoven und zusätzlichen Komponenten.

### Liste der verwendeten Bezugszeichen

- 1: Anlage
- 2: Hauptlinie
- 3: Tabletttransportsystem
- 4: Zuführlinie, erste
- 5: Zuführlinie, zweite
- 6: Zuführlinie, dritte
- 7: Fertigteilübernahme
- 8: lineare Führung
- 9: Tablett
- 10: Auflagefläche
- 11: Maschinenrichtung
- 12: Zuführ-Maschinenrichtung
- 13: Erste Position
- 14: Folgende Position
- 15: Weiter folgende Position
- 16: Trennschnitt
- 17: Folienabschnitt
- 18: Endlosfolienband
- 19: Loch
- 20: Abstand
- 21: Zwischenraum
- 22: Zwischenraum
- 23: Ende der Hauptlinie
- 24: Ostomiebeutel
- 25: Folienabwicklung
- 26: Station
- 27: Station
- 28: Station
- 29: Tablett, bereitstehend
- 30: Halbzeug
- 31: Tablett, in Warteposition
- 32: Station
- 33: Station
- 34: Station
- 35: Station
- 36: Station
- 37: Station
- 38: Station
- 39: Station
- 40: Station
- 41: Entnahme
- 42: Lift
- 43: Greifeinrichtung
- 44: Greifer
- 45: Greifer
- 46: Zentrierpins

## Patentansprüche

1. Anlage (1) zum Herstellen eines Medizinprodukts in Gestalt eines Ostomiebeutels (24), insbesondere eines Kolostomiebeutels, aus mehreren Lagen Folie und/oder Nonwoven,
mit einem Halbzeugtransportsystem, insbesondere einem Tabletttransportsystem (3), mit einer Hauptlinie (2) und mehreren seitlich angeordneten Zuführlinien 4, 5, 6), wobei Halbzeugzuführungen vorgesehen sind, um vorbearbeitete Halbzeuge (30) von den Zuführlinien (4, 5, 6) der Hauptlinie (2) automatisiert zuzuführen,
mit Zuführlinienwerkzeugen an den Zuführlinien (4, 5, 6), und mit einem Hauptwerkzeug an der Hauptlinie (2),
***dadurch gekennzeichnet*, *dass***
die Halbzeugzuführungen modular am Tabletttransportsystem anordenbar sind.

2. Anlage (1)
nach Anspruch 1,
***dadurch gekennzeichnet*, *dass*** die Halbzeugzuführung eine Spreizeinrichtung für das zuzuführende Halbzeug (30) aufweist.

3. Anlage nach einem der vorstehenden Ansprüche, ***dadurch gekennzeichnet*, *dass*** die Hauptlinie ein Hauptschweißwerkzeug aufweist.

4. Anlage nach einem der vorstehenden Ansprüche, ***dadurch gekennzeichnet*, *dass*** eine Zuführlinie ein Vorschweißwerkzeug aufweist.

5. Anlage nach einem der vorstehenden Ansprüche, ***dadurch gekennzeichnet*, *dass*** eine Zuführlinie ein Vorstanzwerkzeug aufweist.

6. Anlage nach einem der vorstehenden Ansprüche, ***dadurch gekennzeichnet*, *dass*** die Halbzeugzuführung voneinander motorisch entfernbare Greifer aufweist.

7. Anlage nach einem der vorstehenden Ansprüche, ***dadurch gekennzeichnet*, *dass*** die Halbzeugzuführung vier Greifer aufweist.

8. Anlage nach einem der vorstehenden Ansprüche, ***dadurch gekennzeichnet*, *dass*** die Halbzeugzuführung an Ecken des Halbzeugs anzugreifen vorgesehene Greifer aufweist.

9. Anlage nach einem der vorstehenden Ansprüche, ***dadurch gekennzeichnet*, *dass*** die Halbzeugzuführung Greifer in Form von Nadeln aufweist.

10. Anlage nach einem der vorstehenden Ansprüche, ***dadurch gekennzeichnet*, *dass*** die Halbzeugzuführung Ablagemittel für das Halbzeug in gespanntem Zustand aufweist, um diese auf Spannungshaltern der Hauptlinie am Halbzeugtransportsystem zu platzieren

11. Anlage nach einem der vorstehenden Ansprüche, ***dadurch gekennzeichnet*, *dass*** alle Halbzeugzuführungen auf identischer Höhe zu greifen und/oder abzulegen eingerichtet sind.

12. Anlage nach einem der vorstehenden Ansprüche, ***dadurch gekennzeichnet*, *dass*** die Anlage Tabletts in Maschinenrichtung auf genau einer Ebene zu führen eingerichtet ist.

13. Anlage nach einem der vorstehenden Ansprüche, ***dadurch gekennzeichnet*, *dass*** zwischen zwei Halbzeugzuführungen eine Warteposition vorgesehen ist.

14. Anlage nach einem der vorstehenden Ansprüche, ***dadurch gekennzeichnet*, *dass*** eine Halbzeugzuführung eine Schlechtteilauszweigung aufweist.

15. Anlage nach einem der vorstehenden Ansprüche, ***dadurch gekennzeichnet*, *dass*** die Hauptlinie eine lineare Transportrichtung aufweist.

16. Anlage nach einem der vorstehenden Ansprüche, ***dadurch gekennzeichnet*, *dass*** vier Lagen zum Herstellen des Produkte miteinander verbunden werden.

17. Anlage nach einem der vorstehenden Ansprüche, ***dadurch gekennzeichnet, dass*** das Halbzeugtransportsystem mit den Halbzeugzuführungen gleichgetaktet ist.

18. Verfahren zum Betreiben einer Anlage nach einem der vorstehenden Ansprüche zum Herstellen eines Medizinprodukts.

19. Verfahren nach Anspruch 18, ***dadurch gekennzeichnet*, *dass*** ein Produktwechsel dadurch vorgenommen wird, dass eine oder mehrere Zuführlinien ausgetauscht werden.
